# EUROPEAN PATENT SPECIFICATION

(11) **EP 2 130 836 B1**
(45) Date of publication and mention of the grant of the patent: **13.02.2013**
(21) Application number: 08010130.6
(22) Date of filing: 03.06.2008
(51) Int. Cl.: C07K 1/107, C07K 14/47, G01N 33/68

(54) **Means and methods for producing zinc fingers and concatemers thereof**
Mittel und Verfahren zur Herstellung von Zinkfingern und Konkatemere daraus
Supports et procédés de production de doigts de zinc et ses concatémères

(43) Date of publication of application: 09.12.2009
(73) Proprietor: Fraunhofer-Gesellschaft zur Förderung der angewandten Forschung e.V., 80686 München (DE)
(72) Inventor: von Nickisch-Rosenegk, Markus, 66386 St. Ingbert (DE); Bier, Frank, 66386 St. Ingbert (DE); Andresen, Heiko, 10713 Berlin (DE)
(74) Representative: Vossius & Partner

(56) References cited:
- WO-A-00/42219
- WO-A-02/08286
- WO-A-02/42459
- WO-A-98/53058
- WO-A-99/06551
- WO-A-99/47656
- WO-A-02/099084
- WO-A-03/062455
- WO-A-03/073105
- WO-A-03/095486
- WO-A-2006/103106
- VON NICKISCH-ROSENEGK MARKUS ET AL: "Chemically synthesized zinc finger molecules as nano-addressable probes for double-stranded DNAs" JOURNAL OF NANOBIOTECHNOLOGY, BIOMED CENTRAL, LONDON, GB, vol. 3, no. 1, 29 June 2005 (2005-06-29), page 5, XP021008671 ISSN: 1477-3155
- FUTAKI S ET AL: "Total synthesis of artificial zinc-finger proteins: Problems and perspectives" BIOPOLYMERS, NEW YORK, NY, US, vol. 76, no. 2, 1 January 2004 (2004-01-01), pages 98-109, XP002357845 ISSN: 0006-3525
- BELIGERE G S ET AL: "Synthesis of a three zinc finger protein, Zif268, by native chemical ligation" BIOPOLYMERS, NEW YORK, NY, US, vol. 51, no. 5, 1 January 1999 (1999-01-01), pages 363-369, XP002357844 ISSN: 0006-3525
- NOMURA WATARU ET AL: "Design and synthesis of artificial zinc finger proteins." METHODS IN MOLECULAR BIOLOGY (CLIFTON, N.J.) 2007, vol. 352, 2007, pages 83-93, XP009107149 ISSN: 1064-3745
- NEGI SHIGERU ET AL: "New redesigned zinc-finger proteins: design strategy and its application." CHEMISTRY - A EUROPEAN JOURNAL, vol. 14, no. 11, 7 April 2008 (2008-04-07), pages 3236-3249, XP009107156 WILEY - V C H VERLAG GMBH & CO. KGAA, WEINHEIM, DE ISSN: 0947-6539
- KAMIUCHI T ET AL: "ARTIFICIAL NINE ZINC-FINGER PEPTIDE WITH 30 BASE PAIR BINDING SITES" BIOCHEMISTRY, AMERICAN CHEMICAL SOCIETY, EASTON, PA.; US, vol. 37, no. 39, 29 September 1998 (1998-09-29), pages 13827-13834, XP000881873 ISSN: 0006-2960
- PAPWORTH ET AL: "Designer zinc-finger proteins and their applications" GENE, ELSEVIER, AMSTERDAM, NL, vol. 366, no. 1, 17 January 2006 (2006-01-17), pages 27-38, XP005282076 ISSN: 0378-1119

## Description

This invention relates to methods of producing zinc finger peptides and concatemers thereof as well as peptides of use in these methods. In particular, the invention relates to peptides consisting of the sequence set forth in any one of SEQ ID NOs: 6 or 8. Furthermore provided are methods of producing a zinc finger domain and concatemers thereof.

Zinc fingers are peptides or small proteins which occur naturally in biological systems. They are capable of specifically binding to double-stranded DNA. Frequently they are involved in controlling the synthesis of proteins at the level of transcription. Zinc fingers as such are well known in the art; see, for example, the corresponding entries in the protein domain data base Pfam maintained at the Sanger Institute and reviewed in the annual data base issues of Nucleic Acids Research; see, for example, Finn et al. (2008). The classical zinc finger domain is the Cys₂His₂ type (Sugiura (2001)). A single zinc finger is capable of specifically recognizing a base triplet. Sugiura (2001) also discusses engineered zinc fingers. In this regard, it has been envisaged to use concatemers of a plurality of zinc finger domains. Such constructs are considered useful in the sequence-specific recognition of longer stretches of double stranded DNA.

Furthermore, it has been discovered that the interaction of zinc fingers with DNA follows a certain code, i.e., there is complementarity between DNA sequences and a certain region of the C2H2 domain (Choo and Klug (1994)). In view of this code, ectopic expression of nucleic acids encoding zinc fingers in cells and organisms has been considered by numerous research teams for various purposes, including control of transcription.

So far, the introduction of non-naturally occurring zinc fingers in cells and organisms generally made use of recombinant nucleic acids which are to be transfected into the target cells or organisms. This approach has several disadvantages in that creation of libraries of zinc fingers complementary to any target sequence is rather cumbersome by means of recombinant DNA technology. Furthermore, and at least in certain settings, the introduction of foreign nucleic acid into cells or organisms poses risks which are desirable to be avoided.

In order to address these concerns, the present inventors at an earlier stage provided means and methods for synthesizing zinc fingers by means of chemical synthesis of peptides (EP1863837). This approach requires a variety of different peptides or libraries of peptides.

WO 2006/103106 describes synthetic zinc finger peptide ligature for forming binding proteins configured in series for addressing specific regions of double-stranded DNA. Von Nickisch-Rosenegk (Journal of Nanobiotechnology (2005), 3 : 5) describes chemically synthesized zinc finger molecules as nano-addressable probes for double-stranded DNAs. WO 02/028286 and WO 03/062455 describe a zinc finger domain recognition code and uses thereof. WO 02/099084 describes composite binding polypeptides. Negi et al. (Chem.Eur.J. (2008), 14:3236-3249) describes new redesigned zinc finger proteins. WO 03/095486 describes the use of a peptide derived from the β-subunit of HCG in order to generate an anti-tumor CTL-type response. WO 98/53058 and WO 99/47656 describe nucleic acid binding proteins. WO 00/42219 describes the selection of sites for targeting by zinc finger proteins as well as methods of designing zinc finger proteins to bind preselected sites. WO 02/42459 describes the position-depending recognition of GNN nucleotide triplets by zinc fingers. WO 03/073105 describes the use of PLAG1 and PLAGL2 in cancer diagnosis and drug screening. WO 99/06551 describes 5' ESTs for secreted proteins identified from brain tissues. Kamiuchi et al. (Biochemistry (1998), 37 : 13827-13834) describes an artificial 9 zinc finger peptide with 30 base pair binding sites. Papworth et al. (Gene (2006), 366 : 27-38) describes designer zinc finger proteins and their applications.

The technical problem underlying the present invention was to provide alternative or improved means and methods for synthesizing zinc fingers.

The present invention is defined by the claims.

Accordingly, the present invention relates to the use of a peptide consisting of the sequence set forth in any one of (a) SEQ ID NOs: 1 to 3; and/or (b) SEQ ID NO: 7 in the synthesis of a peptide, polypeptide or protein, said peptide, polypeptide or protein comprising or consisting of one or more zinc finger domains. Also provided is a peptide consisting of the sequence set forth in any one of SEQ ID NOs: 6 or 8.

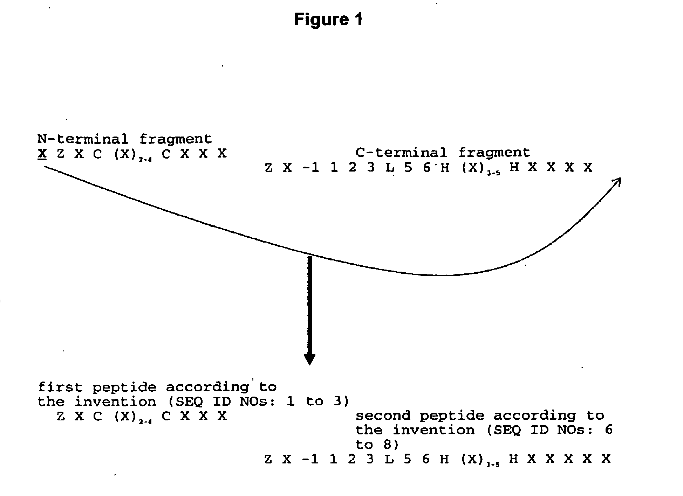

Also described is a peptide consisting of the sequence set forth in any one of SEQ ID NOs: 1 to 3. The peptide consists of the sequence of SEQ ID NO: 1 or of the sequence of SEQ ID NO: 2 or of the sequence of SEQ ID NO: 3. This peptide is also referred to as "first peptide" according to the invention. The sequence of said first peptide is also shown in Figure 1, left hand side, bottom. Preferred is the sequence of SEQ ID NO: 2.

The sequences of SEQ ID NOs: 1 to 3 - and also the sequences of SEQ ID NOs: 6 to 8; see below - are the result of several considerations relating to the synthesis of a single zinc finger domain from two peptides and furthermore relating to the synthesis of concatemers of a plurality of zinc finger domains. The terms "zinc finger domain" and "concatemer" according to the invention are defined further below.

To explain further, the number and variety of building blocks to be used in the synthesis of zinc finger domains and concatemers thereof can be significantly reduced if one considers a single zinc finger domain as consisting of an N-terminal and a C-terminal fragment, wherein the particular choice of the splitting site is of relevance. The specific splitting according to the invention is shown below:
The consensus sequence of the naturally occurring C2H2 domain is shown below:
   X Z X C (X)₂₋₄ C X X X Z X -1 1 2 3 L 5 6 H (X)₃₋₅ H X X X X

The splitting according to the invention is as follows:
X Z X C (X)₂₋₄ C X X X Z X-1 1 2 3 L 5 6 H (X)₃₋₅ H X X X X

X designates any amino acid, Z designates a hydrophobic amino acid selected from L, I, V, M, F, Y, W and C. L, I, V, M, F, Y, W, C as well as H are single letter code abbreviations of the amino acids and accordingly designate Leu, Ile, Val, Met, Phe, Tyr, Trp, Cys and His, respectively. The numbers -1, 1, 2, 3, 5 and 6 designate positions which are relevant for the recognition of a nucleic acid sequence. Position - 1, 1, 2, 3, 5 and 6 may be any amino acid.

The splitting according to the invention in turn has various advantages. First, the zinc finger domain is divided into an N-terminal fragment the sequence of which may remain constant in the envisaged applications, and a C-terminal fragment which comprises those amino acid residues which are responsible for sequence specific binding to double stranded nucleic acids, preferably double stranded DNA.

A further aspect of the splitting is that the N-terminal amino acid of the C-terminal fragment (designated as "Z" in the scheme above) is a hydrophobic amino acid selected from L, I, V, M, F, Y, W and C. In preferred embodiments, this N-terminal amino acid of the C-terminal fragment is Cys (C). Further advantages of the splitting according to the invention which are related to said preferred embodiments are discussed further below.

A further advantage of the splitting is the following: As discussed in conjunction with preferred embodiments below, the N-terminal fragment, more specifically, the first peptide according to the invention, may be labeled in order to allow or facilitate detection of binding to a target sequence of the zinc finger domain or a concatemer thereof according to the invention. By splitting the zinc finger domain into a fragment which does not contain any amino acid residues involved in sequence specific binding, and a second fragment which is involved in sequence specific binding, the number of labeled peptides can be significantly reduced. It is no longer necessary to provide labeled peptides for any base triplet to be recognized.

A further necessary modification of the above described N- and C-terminal fragments in order to arrive at the sequences of SEQ ID NOs: 1 to 3 and 6 to 8 according to the invention is illustrated in Figure 1 enclosed herewith. Taking account of the fact that a single zinc finger domain recognizes a base triplet, it is desirable to produce concatemers of zinc finger domains in order to obtain constructs which are capable of recognizing longer nucleic acid sequences. Having regard to methods of producing concatemers according to the invention as disclosed further below, it is advantageous to remove the N-terminal residue of the N-terminal fragment shown above and attach it to the C-terminus of the C-terminal fragment shown above; see Figure 1. Disregarding the ends of the concatemer to be synthesized, this shift has no influence on the sequence of the individual zinc finger domains within the concatemer. However, such a shift has the advantage that the N-terminal amino acid residue of the obtained first peptide (bottom part of Figure 1) is a hydrophobic amino acid selected from L, I, V, M, F, Y, W and C, preferably C. If the N-terminal residue of the first peptide according to the invention is C, the ligation reactions to be performed in the course of concatemers are further facilitated (see below).

The sequence of said peptide may be the sequence set forth in SEQ ID NO: 4 (CMCTSYCGKR).

The described peptide peptide may comprise one or more labels.

The term "label" is known in the art and refers to any moiety which facilitates detection. Preferred are labels selected from fluorescent, luminescent and radioactive labels.

In a preferred embodiment, the fluorescent label (or fluorophore) is selected from the group consisting of rhodamines, fluorenes, pyrenes, xanthenes including fluoresceines, coumarins, naphthylamines, acridines, benzoxazoles, benzodioxazoles, stilbenes, poly(p-phenylene vinylene)s, polythiophenes, poly(phenylene ethynylene)s and poly(para-phenylene)s. Naphthylamines may have the amino group in the alpha or beta position and include 1-dimethylaminonaphthyl-5-sulfonate, 1-anilino-8-naphthalene sulfonate and 2-p-toluidinyl-6-naphthalene sulfonate. Coumarins include 3-phenyl-7-isocyanatocoumarin ; acridines include 9-isothiocyanatoacridine and acridine orange; and benzoxazoles include N-(p-(2-benzoxazolyl)phenyl)maleimide.

It is understood that the use of the plural form, such as "fluorenes", indicates that not only fluorene itself, but also derivatives thereof known in the art are embraced:

Specific fluorophores according to the invention include the following commercially available (for example from Synthegen) fluorescent dyes: Rhodamine, Tamra-dT, 5-Fluorescein (FITC), 5-Carboxyfluorescein (FAM), 6-Carboxyfluorescein (FAM), 3' 6-Carboxyfluorescein (FAM), 6-Carboxyfluorescein-DMT (FAM-X), 5(6)-Carboxyfluorescein (FAM), 6-Hexachlorofluorescein (HEX), 6-Tetrachlorofluorescein (TET), JOE, LightCycler Red 640, LightCycler Red 705, FAR-Fuchsia (5'-Amidite), FAR-Fuchsia (SE), FAR-Blue (5'-Amidite), FAR-Blue (SE), FAR-Green One (SE), FAR-Green Two (SE), Oregon Green 488, Oregon Green 500, Oregon Green 514, BODIPY FL-X, BODIPY FL, BODIPY-TMR-X, BODIPY R6G, BODIPY 650/665, BODIPY 564/570, BODIPY 581/591, BODIPY TR-X, BODIPY 630/650, BODIPY 493/503, Carboxyrhodamine 6G, MAX, 5(6)-Carboxytetramethylrhodamine (TAMRA), 6-Carboxytetramethylrhodamine (TAMRA), 5(6)-Carboxy-X, Rhodamine (ROX), 6-Carboxy-X-Rhodamine (ROX), AMCA-X (Coumarin), Texas Red-X, Rhodamine Red-X, Marina Blue, Pacific Blue, Rhodamine Green-X, 7-diethylaminocoumarin-3-carboxylic acid, 7-methoxycoumarin-3-carboxylic acid, Cy3, Cy3B, Cy5, Cy5.5, DY-505, DY-550, DY-555, DY-610, DY-630, DY-633, DY-636, DY-650, DY-675, DY-676, DY-681, DY-700, DY-701, DY-730, DY-750, DY-751, DY-782, Cy3.5 and EDANS. A further preferred fluorescent label according to the invention are quantum dots.

Preferred radioactive labels include ³²P, ³⁵S and ³H.

Labeled forms of peptides according to the invention are useful, for example, when said peptides are to be used as probes for the specific detection of nucleic acid molecules. The term "probe" is known in the art and refers to molecules or moieties which are capable of binding, preferably specifically binding to target molecules such as nucleic acids. As discussed herein above, zinc finger domains as well as concatemers thereof are capable of specifically recognizing base sequences of nucleic acids.

Said label may be attached to to the the N-terminus.

The label may either be attached to the N-terminal amino acid of said peptide or to an amino acid residue which has previously been added to the N-terminus of said peptide. A preferred embodiment of such an additional amino acid residue is Gly.

The above disclosed peptide may further comprise at the N-terminus a membrane-penetrating (poly)peptide. Membrane-penetrating (poly)peptides are known in the art; see, for example, Zorko, M and Langel, U. (2005), Advanced Drug Delivery Reviews; 57: 529-545.

In case both a label and a membrane-penetrating (poly)peptide are attached to the first peptide according to the invention, said label may be either attached to any amino acid of the sequence of any one of SEQ ID NOs: 1 to 3 or to any amino acid of said membrane-penetrating (poly)peptide. In preferred embodiments, said label is attached to the N-terminal amino acid of said membrane-penetrating (poly)peptide. Furthermore, it is envisaged to attach more than one label to first peptides according to the invention.

The term (poly)peptide embraces both peptides and polypeptides. The term "peptide" as used herein refers to polymers of amino acids consisting of up to 30 amino acids. The term "polypeptide" refers to polymers of amino acids consisting of more than 30 amino acids.

In many cases, crossing of biological membranes can be achieved or enhanced by conjugating protein transduction domains (or protein transducing domains; PTDs) to a target molecule. Here, the term "target molecule" includes peptides, zinc finger domains and concatemers according to the invention. PTDs are generally short to medium size (poly)peptide sequences, for this reason often called cell-penetrating (poly)peptides (CPPs) or membrane-penetrating (poly)peptides, and do not exhibit marked lipophilicity, but are amphipathic and generally carry a number of positive charges at physiological pH. They are able to efficiently cross the plasma membrane of cells, either alone, or linked to a 'cargo'.

Said membrane-penetrating (poly)peptide may be selected from the group consisting of peptides containing the sequence motif Penetratin (Antennopedia 43-58), TaT, TAT 46-60, PTD-4, SynB1 and SynB3 (Protegrin I), Transportan, MAP (Model Amphipatic peptide), Pep-1, Proline rich peptide (VXLPPP)ₙ, pVEC, piSL, polyArginine, SN50, MPG; see Zorko, M and Langel, U. (2005), Advanced Drug Delivery Reviews; 57: 529-545. The above mentioned membrane-penetrating peptides can be in the natural form (formed by L-residues), or formed by D-residues.

A membrane-penetrating peptide is the sequence set forth in SEQ ID NO: 5 (GRKKRRQRRR), also known in the art as a TAT-motif.

The membrane-penetrating (poly)peptide may be fused to the N-terminus of the peptide according to the invention by a normal peptide bond. Alternatively, an intervening sequence is present which is preferably 1 to 5, more preferred 3 to 5 residues in length. Said intervening sequence may comprise or consist of Gly residues. The N-terminus of the intervening sequence may be fused to the C-terminus of said membrane-penetrating (poly)peptide via a normal main chain peptide bond. Also, the C-terminus of the intervening sequence may be fused to the N-terminus of the sequence of any one of SEQ ID NOs: 1 to 3 via a normal main chain peptide bond. A corresponding first peptide of the invention has accordingly the sequence: membrane-penetrating (poly)peptide - intervening sequence - sequence of any one of SEQ ID NOs: 1 to 3.

The terms "normal peptide bond" or "main chain peptide bond" as used herein refers to an amide bond (-C(=O)-NH-) between the carboxylate attached to the α-carbon of one amino acid and the amino group attached to the α-carbon of the other amino acid.

Upon translocation across the plasma membrane, a zinc finger domain or a concatemer thereof according to the present invention, wherein said zinc finger domain or concatemer comprises at the very N-terminal a first peptide according to the invention as described above, wherein said peptide in turn carries at its N-terminus a membrane-penetrating (poly)peptide, can freely diffuse within the cell and enter the nucleus. The pores of the nucleus have a cut of at about 40 kD. Accordingly, it is preferred that concatemers according to the invention which are described further below have a molecular weight below 40 kD. Preferably, they do not comprise more than about 300 amino acids.

It is envisaged to either use a first peptide according to the invention which is conjugated to a membrane penetrating (poly)peptide as a building block in the synthesis of a zinc finger domain or concatemer according to the invention, or to synthesize the zinc finger domain or concatemer thereof and subsequently fuse a membrane-penetrating polypeptide to the N-terminus of the obtained zinc finger domain or concatemer thereof. "Fusing" as used herein refers to the formation of a main chain peptide bond. In the latter case, ligation methods as described in more detail further below may be used.

As an alternative to the above described membrane-penetrating peptides, one may use constructs which bind to cell surface molecules and are subsequently internalized. Suitable constructs are (poly)peptides comprising a motif consisting of the three amino acids RGD, which are known to bind to integrin and accordingly are also capable of carrying a cargo into the cell. The considerations relating to the conjugation of membrane-penetrating (poly)peptides the constructs according to the invention apply *mutatis mutandis* to (poly)peptides comprising an RGD motif.

Furthermore, it is envisaged to conjugate peptides, zinc finger domains and concatemers according to the invention with any other moiety which confers the capability to cross cell membranes.

The present invention furthermore provides a peptide consisting of the sequence set forth in any one of SEQ ID NOs: 6 and 8. This peptide is also referred to as "second peptide" according to the invention. The sequence of said second peptide is also shown in Figure 1, right hand side, bottom. Preferred is the sequence of SEQ ID NO: 6.

Advantages of a peptide consisting of the sequence of any one of SEQ ID NOs: 6 to 8 are discussed herein above in conjunction with the peptide consisting of the sequence of any one of SEQ ID NOs: 1 to 3.

First and second peptides according to the invention are obtainable via any suitable synthesis method including synthesis from the individual amino acid monomers. Methods of synthesizing peptides from amino acids are well-known in the art and described, for example, in Pipkorn et al. (2002).

The present invention furthermore provides a collection of peptides, the collection comprising or consisting of a plurality of different peptides, each peptide consisting of a sequence as set forth in any one of SEQ ID NOs: 1, 2, 3, 6, 7 or 8.

The term "collection" refers to a plurality of entities, in the context of this embodiment of peptides. The term "collection" according to the invention includes "library".

This collection, to the extent reference is made to the sequence of any one of SEQ ID NOs: 1 to 3, as well as preferred embodiments thereof, is referred to as "collection of first peptides" according to the invention. This collection, to the extent reference is made to the sequence of any one of SEQ ID NOs: 6 to 8, as well as preferred embodiments thereof, is referred to as "collection of second peptides" according to the invention. Accordingly, provided are collections consisting of peptides, each peptide consisting of a sequence as set forth in any one of SEQ ID NOs: 1 to 3. Also provided are collections consisting of peptides, each peptide consisting of a sequence as set forth in any one of SEQ ID NOs: 6 to 8. Furthermore, provided are collections consisting of peptides, wherein peptides consisting of a sequence as set forth in any one of SEQ ID NOs: 1 to 3 are included as well as peptides consisting of a sequence as set forth in SEQ ID NOs: 6 to 8. Further peptides, which neither consist of a sequence of any one of SEQ ID NOs: 1 to 3 nor of a sequence of any one of SEQ ID NOs: 6 to 8, may also be comprised in collections according to the invention.

Said collection of second peptides has the advantage that each of the peptides, once being part of a zinc finger domain, is capable of performing sequence specific recognition of nucleic acids. A suitably designed collection, which is subject of a more preferred embodiment discussed further below, comprises peptide sequences permitting recognition of all 64 base triplet sequences formed by the 4 nucleobases.

In a preferred embodiment of the peptides consisting of the sequence set forth in any one of SEQ ID NOs: 6 to 8 or of the collection of peptides described above, the N-terminal residue of the sequence of any one of SEQ ID NOs: 6 to 8 in said peptide or in at least one of said peptides is Cys.

An N-terminal Cys permits the use of a specific ligation method which is discussed further below.

In a further preferred embodiment, the collection comprises or consists of all peptides consisting of the sequences set forth in SEQ ID NOs: 9 to 72.

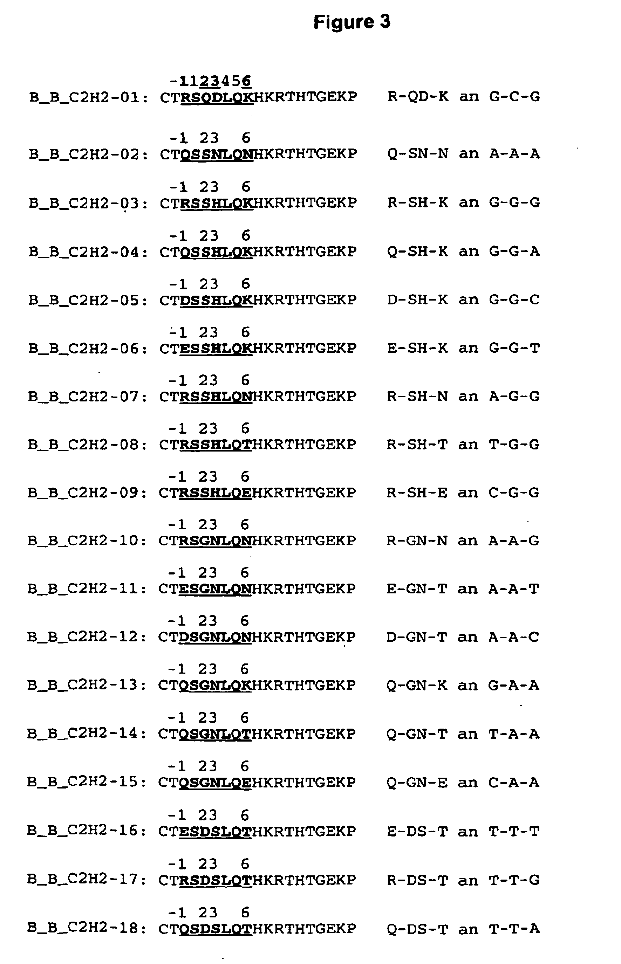

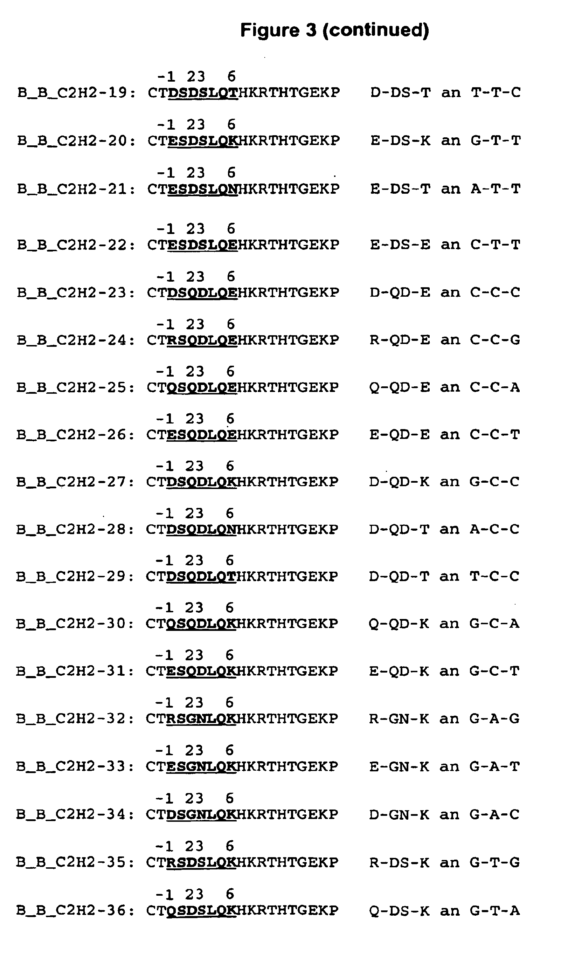

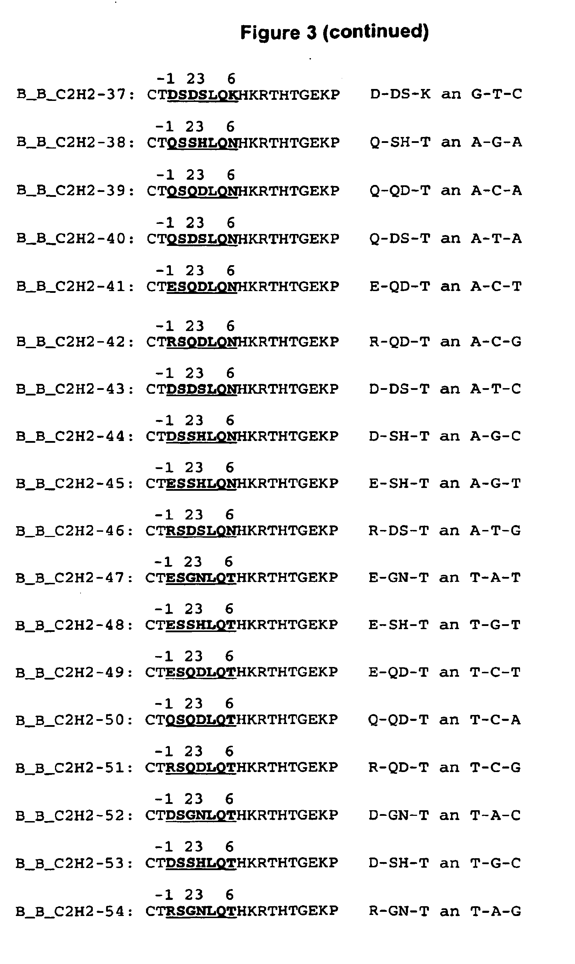

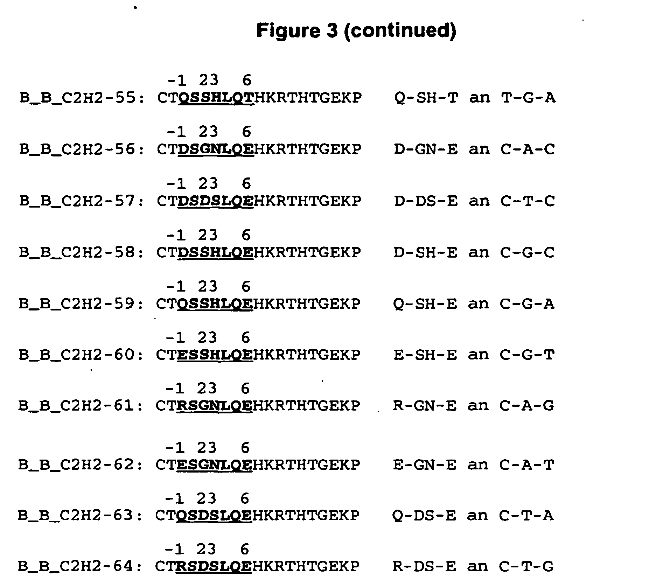

A compilation of the sequences of SEQ ID NOs: 9 to 72 is also provided in Figure 3 enclosed herewith. Figure 3 furthermore displays the base triplet being recognized by the respective peptide sequence.

In preferred embodiments of the peptides according to the invention or of the collection according to the invention, the C-terminal residue of said peptide or the C-terminal residues of at least one peptide of said collection carries a thioester. A C-terminal thioester has implications for the ligation strategy to be used; for details see below.

Described is furthermore a peptide consisting of the sequence set forth in any one of SEQ ID NOs: 73 to 81.

The sequence set forth in any one of SEQ ID NOs: 73 to 81 is a generic sequence of the zinc finger domain according to the present invention. When reference is made to a "zinc finger domain" according to the invention, a peptide consisting of the sequence of any one of SEQ ID NOs: 73 to 81 is intended. The sequence of any one of SEQ ID NOs: 73 to 81 is the result of the ligation of a peptide consisting of a sequence set forth in any one of SEQ ID NOs: 1 to 3 as the N-terminal component with a peptide consisting of a sequence set forth in any one of SEQ ID NOs: 6 to 8 as the C-terminal component. Suitable ligation methods are discussed further below.

The term "N-terminal component" as used herein designates an amino acid or peptide which provides the carboxylate for ligation. The term "C-terminal component" as used herein designates an amino acid or peptide which provides the amino group for ligation. "Ligation" as used herein in conjunction with amino acids, peptides, polypeptides or proteins refers to the formation of a main chain peptide bond between the carboxylate of the C-terminal amino acid of an N-terminal component and the α-amino group of the N-terminal amino acid of a C-terminal component.

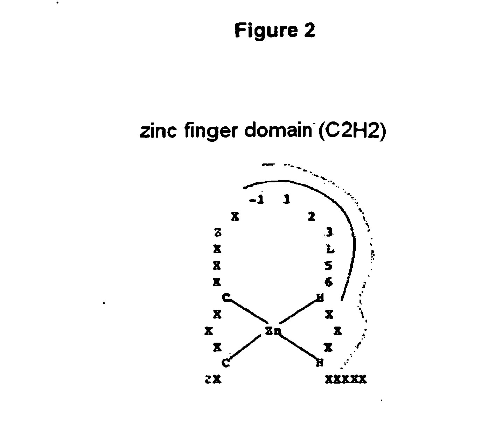

The specific domain boundaries, i.e., the respective N- and C-termini, of the sequence of any one of SEQ ID NOs: 73 to 81 are the result of the considerations illustrated in Figure 1 enclosed herewith. Figure 2 presents a schematic drawing of the structure of an exemplary zinc finger domain according to the invention.

Also described is a collection peptides, each peptide consisting of a sequence as set forth in any one of SEQ ID NOs: 71 to 83.

In a preferred embodiment of said collection, each peptide is the result of the ligation of a peptide consisting of a sequence set forth in any one of SEQ ID NOs: 1 to 3 as the N-terminal component with a second peptide according to the invention chosen from peptides consisting of the sequence as set forth in SEQ ID NOs: 9 to 72 as the C-terminal component. In a further preferred embodiment, at least 64 different peptides are comprised in said collection.

Each of the second peptides consisting of a sequence set forth in any one of SEQ ID NOs: 9 to 72 may be comprised in a peptide of said collection.

Said first peptide being the N-terminal component of the peptides of said collection may consist of the sequence set forth in SEQ ID NO: 4.

The present invention furthermore provides a concatemer of peptides, each peptide consisting of a sequence set forth in any one of SEQ ID NOs: 73 to 81.

When reference is made herein to a concatemer according to the invention, it is intended to refer to a concatemer of peptides, wherein each peptide consists of a sequence set forth in any one of SEQ ID NOs: 73 to 81. The linkages between the constituent zinc finger domains within a concatemer according to the invention are normal main chain peptide bonds. Using the designation system of Figures 1 and 2, a concatemer according to the invention can be represented as follows:
(Z X C (X)₂₋₄ C X X X Z X -1 1 2 3 L 5 6 H (X)₃₋₅ H X X X X X)ₙ

Preferred values of n are 2, 3, 4, 5, 6, 7, 8, 9 and 10. Particularly preferred values are 6, 7 and 8. The human genome consists of about 3 billion bases. The minimal length of an oligonucleotide to be able to uniquely recognize a sequence within the human genome is generally considered to be 16 bases, noting that more than 4 billion different oligonucleotides having a length of 16 bases can be constructed using the 4 nucleobases. A corresponding concatemer would have to consist of at least 6 zinc finger domains, noting that a single zinc finger domain recognizes a sequence of three contiguous bases. Furthermore, it is known that contiguous zinc finger domains within a concatemer recognize contiguous base triplets; see, for example, Sugiura, *loc. cit.*

Described is furthermore a collection of concatemers, each concatemer being defined herein above. Considerations relating to the diversity of a single zinc finger domain according to the invention as detailed herein above apply *mutatis mutandis* to the collection of concatemers according to the invention. Since the sequence of each zinc finger domain according to the invention within a concatemer according to the invention may be chosen independently, a vast number of different concatemer sequences is provided.

The present invention furthermore relates to a method of producing a peptide consisting of the sequence set forth in any one of SEQ ID NOs: 73 to 81, the method comprising the step of ligating a first peptide according to the invention as the N-terminal component with a second peptide according to the invention as the C-terminal component. The terms "first peptide" and "second peptide" are defined herein above. Also provided is the method of producing a collection of peptides, each peptide being a peptide consisting of the sequence set forth in any one of SEQ ID NOs: 73 to 81, the method comprising the step of ligating one or more first peptides according to the invention as the N-terminal component(s) with one or more second peptides according to the invention or with a collection according to the invention as the C-terminal component(s). Accordingly, the methods of producing according to the invention may make use of a single species as the N-terminal component and/or of a single species as the C-terminal component. Alternatively, either one or both components may be provided as a mixture of distinct molecular species.

Said one or more first peptides may consist of the sequence of SEQ ID NO: 4, and/or said one or more second peptides may be chosen from the collection of the second peptides according to the invention as disclosed herein above.

The term "ligating" as used in conjunction with peptides according to the invention refers to the formation of a normal main chain peptide bond between the C-terminal amino acid residue of the N-terminal component with the N-terminal amino acid residue of the C-terminal component.

Peptide ligation methods are known in the art. Initially, methods were developed which require the presence of a thioester at the C-terminal amino acid of the N-terminal component and furthermore a Cys being the N-terminal amino acid of the C-terminal component; see, for example, Hackeng et al. (1999). Later on, more general ligation methods have been developed which do not exhibit these limitations; see, for example, Nilsson et al. (2001), Yeo et al. (2004), and Harwig et al. (2000). David et al. (2004) provides a review of ligation methods.

The present invention furthermore relates to a method of producing a concatemer of peptides, the concatemer consisting of a sequence as set forth in any one of SEQ ID NOs: 73 to 81, the method comprising (a) ligating a first peptide according to the invention as the N-terminal component with a second peptide according to the invention as the C-terminal component; (b) ligating the product obtained in step (a) as the C-terminal component with a second peptide according to the invention as the N-terminal component; (c) ligating the product obtained in step (b) as the C-terminal component with a first peptide according to the invention as the N-terminal component; (d) optionally repeating steps (b) and (c) until a concatemer of desired length is obtained; (e) ligating the product obtained in step (c) or (d), respectively, as the C-terminal component, with a second peptide according to the invention as the N-terminal component; and (f) ligating the product obtained in step (e) as the C-terminal component with a first peptide according to the invention as the N-terminal component.

A "first peptide" may optionally comprise a label and/or a membrane-penetrating (poly)peptide as defined above.

Similarly, the present invention also provides a method of producing a collection of concatemers of peptides, the concatemer consisting of a sequence as set forth in any one of SEQ ID NOs: 73 to 81, the method comprising (a) ligating one or more first peptides according to the invention as the N-terminal components with one or more second peptides according to the invention or with a collection of second peptides according to the invention as the C-terminal components; (b) ligating the products obtained in step (a) as the C-terminal components with one or more second peptides according to the invention or with a collection of second peptides according to the invention, respectively, as the N-terminal components; (c) ligating the products obtained in step (b) as the C-terminal components with one or more first peptides according to the invention as the N-terminal components; (d) optionally repeating steps (b) and (c) until a concatemer of desired length is obtained; (e) ligating the products obtained in step (c) or (d), respectively, as the C-terminal components, with one or more second peptides according to the invention or with a collection of second peptides according to the invention, respectively, as the N-terminal components; and (f) ligating the products obtained in step (e) as the C-terminal components with one or more first peptides according to the invention as the N-terminal components.

In preferred embodiments of the methods of producing according to the invention, (i) the C-terminal residue of the N-terminal component(s) carries a thioester; (ii) the N-terminal residue of the C-terminal component(s) is Cys; and (iii) ligating is performed under conditions permitting the formation of a peptide bond between a C-terminal thioester and an N-terminal thiol-containing amino acid. Suitable conditions are known to the skilled person and further detailed in the references cited herein above.

In these preferred embodiments, to the extent they relate to a method of producing a concatemer according to the invention, it is sufficient to provide the collection of second peptides in two forms. In one form, the C-termini of the second peptides comprised in the collection carry a thioester, and in the other form they do not and instead have a free carboxy terminus. To explain further, in step (a) of the method of producing a collection of concatemers according to the invention, it is envisaged to use a collection of second peptides, wherein the second peptides have a free carboxy terminus. These second peptides eventually become the most C-terminal part of the concatemer to be synthesized, noting that the synthesis of concatemers according to the invention proceeds from the C- to the N-terminus. In the remainder of the steps of the method of producing a collection of concatemers according to the invention, to the extent use is made of a collection of second peptides according to the invention, it is envisaged to use a collection of second peptides, wherein the C-terminal amino acid residues of the second peptides carry a thioester.

Whenever the N-terminal residue of the N-terminal component carrying a thioester is Cys, said Cys is protected during the step of ligating said N-terminal component to a C-terminal component. Thereby cyclisation of said N-terminal component is prevented. Prior to a subsequent step of ligating, if any, the Cys is deprotected and thereby made available for ligation in said subsequent step of ligating. Protection of Cys as used herein refers to protection from forming peptide bonds. Suitable methods are known in the art and include conversion of Cys into thiazolidine (protection) and shifting pH to convert the thiazolidine back into Cys (deprotection); see for example, Bang and Kent (2004).

As regards the first peptide according to the invention, these peptides are always used as N-terminal components. Accordingly, it is envisaged to use always the thioester form. For the final step of the method of producing it is furthermore envisaged to optionally use first peptides which in turn carry a label and/or are conjugated with a membrane-penetrating (poly)peptide.

The present invention furthermore provides the use of a first peptide according to the invention, of a second peptide according to the invention and/or of a collection according to the invention in the synthesis of a peptide, polypeptide or protein, said peptide, polypeptide or protein comprising or consisting of one or more zinc finger domains.

The terms "peptide" and "polypeptide" are defined herein above. In accordance with the invention, the term "polypeptide" comprises "proteins" as long as the proteins consist of a single polypeptide chain. On the other hand, proteins are envisaged which comprise more than a single polypeptide chain.

Described is furthermore the use of a concatemer of peptides, each peptide consisting of a sequence as set forth in any one of SEQ ID NOs. 73 to 81, or the concatemer being obtainable by the methods of producing concatemers or collections of concatemers according to the invention, (a) as sequence-specific nucleic acid-binding probe; and/or (b) as transcription factor or component thereof.

As stated above, any based triplet formed by the four nucleobases can be specifically recognized by a second peptide according to the invention, wherein said second peptide is comprised in a zinc finger domain according to the invention. Accordingly, concatemers of *n* zinc finger domains according to the invention, *n* being an integer number greater than or equal 2, are capable of specifically recognizing a sequence of 3n nucleobases. For this embodiment of the use according to the invention, i.e., the use as a probe, it is preferred to use labeled forms of the concatemers.

Naturally occurring zinc finger domains as well as proteins comprising one or more zinc finger domains are known to function as transcription factors or as components thereof. The term "component" refers to zinc finger domains or concatemers thereof being a domain or a plurality of domains, respectively, within a multi-domain protein. The term "domain" or "protein domain" is well known in the art; see, for example, Finn et al. (2008). Accordingly, it is envisaged to use concatemers according to the invention as artificial transcription factors in the control of the transcription of one or more target genes. The construction of artificial transcription factors based on zinc fingers is described in, for example, Bae et al. (2003).

Accordingly, described is furthermore a concatemer according to the invention for use in gene therapy.

Also provided is a kit comprising or consisting of (a) one or more first peptides according to the invention; and (b) one or more second peptides according to the invention and/or a collection of first and/or second peptides according to the invention.

Such a kit is *inter alia* useful for the methods of producing according to the invention. Accordingly, in a preferred embodiment, the kit further comprises a manual with instructions for performing said methods of producing according to the invention.

The present invention furthermore provides a kit comprising or consisting of (a) a collection of peptides, each peptide consisting of a sequence as set forth in any one of SEQ ID NOs: 73 to 81, or a collection being obtainable by the method of producing a collection of peptides according to the invention; and/or (b) a collection of concatemers of peptides, each peptide consisting of a sequence set forth in any one of SEQ ID NOs: 73 to 81, or a collection being obtainable by the method of producing a collection of concatemers of peptides according to the invention.

Such a kit will *inter alia* be useful as a collection of probes or building blocks of probes, and/or as transcription factors or building blocks of transcription factors.

These applications are to be seen in analogy with present approaches for interfering with gene expression and based on nucleic acid constructs such as siRNAs, micro RNAs and the like.

The present invention furthermore provides a solid support with one or more peptides, one or more concatemers, and/or one or more collections being immobilized on the surface of said support; said peptide(s) consisting of a sequence set forth in any one of SEQ ID NOs: 73 to 81; said concatemers(s) being concatemers(s) of peptides, each peptide consisting of the sequence set forth in any one of SEQ ID NOs: 73 to 81; and said collections consisting of comprising one or more collections of peptides, each peptide consisting of a sequence set forth in any one of SEQ ID NOs: 73 to 81, or the one or more collections of peptides being obtainable by the method of producing a collection of peptides; and/or one or more collections of concatemers, each concatemer being a concatemer of peptides, each peptide consisting of a sequence as set forth in any one of SEQ ID NOs: 73 to 81, or the one or more collections or concatemers being obtainable by the method of producing a collection of concatemers of peptides. Such solid support will *inter alia* be useful in applications which are presently being conducted with microarrays or DNA chips. In this regard it has to be noted that, in order to be amenable to detection by zinc finger domains or concatemers according to the invention, target nucleic acids have to be double stranded. This avoids the necessity of providing target nucleic acids in single stranded form, which is a prerequisite for target nucleic acids when using oligonucleotide probes of the prior art.

Methods of coupling (poly)peptides to solid supports are known in the art and are described in, for example, Andresen et al. (2006).

The figures show:
- **Figure 1:**: Illustration of the design of first and second peptides according to the invention. Illustrated is both splitting according to the invention and the shifting of the N-terminal residue of the N-tenninal fragment to the C-terminus of the C-terminal fragment.
- **Figure 2:**: Schematic representation of a zinc finger domain according to the invention. X stands for any amino acid, Z for a hydrophobic amino acid selected from L, I, V, M, F, Y, W, C as well as H are single letter code abbreviations of the amino acids and accordingly designate Leu, Ile, Val, Met, Phe, Tyr, Trp, Cys and His, respectively. The numbers -1, 1, 2, 3, 5 and 6 designate positions which are relevant for the recognition of a base triplet. The inner curved line extending from residues -1 to 6 designates the region of the zinc finger domain facing the base triplet to be recognized. The outer curved line indicates the region of the zinc finger domain assuming an α-helical confirmation.
- **Figure 3:**: Figure 3 displays the collection of second peptides according to the invention, wherein the collection consists of the peptides consisting of the sequences set forth in SEQ ID NOs. 9 to 72. On the left hand side, an internal designation is provided. In the middle part, the sequence of the respective second peptide is provided together with an indication of the positions -1 to 6 involved in the binding of the base triplet. On the right part, the key residues at positions -1 to 3 and 6 are reproduced as is the sequence of the base triplet being recognized.

### Further references

Ahel et al., Nature 451: 81-85 (2008)
Andresen et al., Journal of Immunological Methods 315, 11-18 (2006)
Bae et al., Nature Biotechnology 21, 275-280 (2003)
Bang et al., Angewandte Chemie International Edition 43: 2534-2538 (2004)
Choo and Klug, Proc. Natl. Acad. 91, 11163-11167 (1994)
David et al., Eur. J. Biochem. 271, 663-677 (2004)
Finn et al., Nucl. Acids Res. 36, D281-D288 (2008)
Hackeng et al., Proc. Natl. Acad. Sci. 96, 10068-10076 (1999)
Harwig C.W. et al., Bioorg. Med. Chem. Lett. 10, 915-918 (2000)
Nilsson et al., Organic Letters 3, 9-12 (2001)
Pipkom et al., Journal of Peptide Research 59, 105 (2002)
Sugiura, RIKEN Review 35, 102-104 (2001)
Yeo et al., Chemistry 10, 4664-72 (2004)

## Claims

1. Use of a peptide consisting of the sequence set forth in any one of
(a) SEQ ID NOs: 1 to 3; and/or
(b) SEQ ID NO: 7
in the synthesis of a peptide, polypeptide or protein, said peptide, polypeptide or protein comprising or consisting of one or more zinc finger domains.

2. The use according to claim 1(a), wherein the N-terminal residue of the sequence of any one of SEQ ID NOs: 1 to 3 is Cys.

3. A peptide consisting of the sequence set forth in any one of SEQ ID NOs: 6 or 8.

4. A collection of peptides, the collection comprising or consisting of a plurality of different peptides, each peptide consisting of a sequence as set forth in SEQ ID NOs: 1, 2, 3, 6, 7 or 8.

5. The use of claim 1 (b), the peptide of claim 3 or the collection of peptides of claim 4, wherein the N-terminal residue of the sequence of any one of SEQ ID NOs: 6 to 8 in said peptide or in at least one of said peptides is Cys.

6. The collection of claim 5, wherein the collection comprises or consists of all peptides consisting of the sequences set forth in SEQ ID NOs: 9 to 72.

7. The use according to claim 1, 2 or 5, the peptide according to claim 3 or 5, or the collection according to any one of claims 4 to 6, wherein the C-terminal residue of said peptide or the C-terminal residues of at least one peptide of said collection carries a thioester.

8. A concatemer of peptides, each peptide consisting of the sequence set forth in any one of SEQ ID NOs: 73 to 81.

9. A method of producing a peptide consisting of the sequence set forth in any one of SEQ ID NOs: 73 to 81, said method comprising the step of ligating a peptide consisting of the sequence set forth in any one of SEQ ID NOs 1 to 3 as the N-terminal component with a peptide consisting of the sequence set forth in any one of SEQ ID NOs: 6 to 8 as the C-terminal component.

10. A method of producing a collection of peptides, each peptide consisting of the sequence set forth in any one of SEQ ID NOs: 73 to 81, the method comprising the step of ligating one or more peptides as defined in claim 1 (a) as the N-terminal component(s) with one or more peptides as defined in claim 1 (b) or 3 or with a collection as defined in claim 4 as the C-terminal component(s).

11. A method of producing a concatemer of peptides, the concatemer being as defined in claim 8, the method comprising
(a) ligating a peptide as defined in claim 1 (a) as the N-terminal component with a peptide as defined in claim 1(b) or 3 as the C-terminal component;
(b) ligating the product obtained in step (a) as the C-terminal component with a peptide as defined in claim 1 (b) or 3 as the N-terminal component;
(c) ligating the product obtained in step (b) as the C-terminal component with a peptide as defined in claim 1 (a) as the N-terminal component;
(d) optionally repeating steps (b) and (c) until a concatemer of desired length is obtained;
(e) ligating the product obtained in step (c) or (d), respectively, as the C-terminal component, with a peptide as defined in claim 1 (b) or 3 as the N-terminal component; and
(f) ligating the product obtained in step (e) as the C-terminal component with a peptide as defined in claim 1 (a) as the N-terminal component.

12. A method of producing a collection of concatemers of peptides, the concatemer being as defined in claim 8, the method comprising
(a) ligating one or more peptides as defined in claim 1 (a) as the N-terminal components with one or more peptides as defined in claim 1 (b) or 3 or with a collection as defined in claim 4 as the C-terminal components;
(b) ligating the products obtained in step (a) as the C-terminal components with one or more peptides as defined in claim 1 (b) or 3 or with a collection as defined in claim 4, respectively, as the N-terminal components;
(c) ligating the products obtained in step (b) as the C-terminal components with one or more peptides as defined in claim 1 (a) as the N-terminal components;
(d) optionally repeating steps (b) and (c) until a concatemer of desired length is obtained;
(e) ligating the products obtained in step (c) or (d), respectively, as the C-terminal components, with one or more peptides as defined in claim 1(b) or 3 or with a collection as defined in claim 4, respectively, as the N-terminal components; and
(f) ligating the products obtained in step (e) as the C-terminal components with one or more peptides as defined in claim 1(a) as the N-terminal components.

13. The method of any one of claims 9 to 12, wherein
(i) the C-terminal residue of the N-terminal component(s) carries a thioester;
(ii) the N-terminal residue of the C-terminal component(s) is Cys; and
(iii) ligating is performed under conditions permitting the formation of a peptide bond between a C-terminal thioester and an N-terminal thiol-containing amino acid.

14. A kit comprising or consisting of
(a) one or more peptides as defined in any one of claims 1 (a) or 2; and
(b) one or more peptides as defined in any one of claims 1 (b), 3, 5 or 7 and/or a collection according to any one of claims 4 to 7.

15. A kit comprising or consisting of
(a) a collection of peptides, each peptide consisting of the sequence set forth in any one of SEQ ID NOs: 73 to 81, or a collection being obtainable by the method of claim 10; and/or
(b) a collection of concatemers, each concatemer being as defined in claim 8, or a collection being obtainable by the method of claim 12.

16. A solid support with one or more peptides, one or more concatemers, and/or one or more collections being immobilized on the surface of said support;
said peptide(s) consisting of the sequence set forth in any one of SEX ID NOs: 73 to 81; said concatemers(s) being as defined in claim 8; and
said collections consisting of or comprising one or more collections of peptides, each peptide consisting of the sequence set forth in any one of SEQ ID NOs: 73 to 81, or the one or more collections of peptides being obtainable by the method of claim 10; and/or one or more collections of concatemers, each concatemer being as defined in claim 8, or the one or more collections of concatemers being obtainable by the method of claim 12.

## Patentansprüche

1. Verwendung eines Peptids bestehend aus der Sequenz wie sie in
(a) SEQ ID Nr.: 1 bis 3; und/oder
(b) SEQ ID Nr.: 7
dargestellt ist, in der Synthese eines Peptids, Polypeptids oder Proteins, wobei das Peptid, Polypeptid oder Protein eine oder mehrere Zinkfingerdomänen umfasst oder aus diesen besteht.

2. Verwendung nach Anspruch 1 (a), wobei der N-terminale Rest der Sequenz einer der SEQ ID Nr.: 1 bis 3 Cys ist.

3. Peptid bestehend aus der Sequenz wie sie in einer der SEQ ID Nr.: 6 oder 8 dargestellt ist.

4. Sammlung von Peptiden, wobei die Sammlung eine Vielzahl verschiedener Peptide umfasst oder aus diesen besteht, wobei jedes Peptid aus einer Sequenz besteht, wie sie in SEQ ID Nr.: 1, 2, 3, 6, 7 oder 8 dargestellt ist.

5. Verwendung nach Anspruch 1 (b), Peptid nach Anspruch 3 oder Sammlung von Peptiden nach Anspruch 4, wobei der N-terminale Rest der Sequenz einer der SEQ ID Nr.: 6 bis 8 in dem Peptid oder in zumindest einem der Peptide Cys ist.

6. Sammlung nach Anspruch 5, wobei die Sammlung alle Peptide, die aus den in SEQ ID Nr.: 9 bis 72 dargestellten Sequenzen bestehen, umfasst oder aus diesen besteht.

7. Verwendung nach Anspruch 1, 2 oder 5, Peptid nach Anspruch 3 oder 5, oder Sammlung nach einem der Ansprüche 4 bis 6, wobei der C-terminale Rest des Peptids oder der C-terminale Rest mindestens eines Peptids der Sammlung einen Thioester trägt.

8. Concatemer von Peptiden, wobei jedes Peptid aus einer Sequenz besteht, wie sie in einer der SEQ !D Nr.: 73 bis 81 dargestellt ist.

9. Verfahren zum Herstellen eines Peptids, das aus der Sequenz besteht, wie sie in einer der SEQ ID Nr.: 73 bis 81 dargestellt ist, wobei das Verfahren den Schritt des Ligierens eines Peptids umfasst, das aus einer in SEQ ID Nr.: 1 bis 3 dargestellten Sequenz besteht, als N-terminale Komponente mit einem Peptid, das aus einer in der SEQ ID Nr.: 6 bis 8 dargestellten Sequenz besteht, als C-terminale Komponente.

10. Verfahren zum Herstellen einer Sammlung von Peptiden, wobei jedes Peptid aus der Sequenz besteht, die in einer der SEQ ID Nr.: 73 bis 81 dargestellt ist, wobei das Verfahren den Schritt des Ligierens eines oder mehrerer Peptide wie in Anspruch 1(a) definiert als N-terminale Komponente(n) mit einem oder mehreren Peptiden wie in Anspruch 1(b) oder 3 definiert oder mit einer Sammlung wie in Anspruch 4 definiert als C-terminale Komponente(n) umfasst.

11. Verfahren zum Herstellen eines Concatemers von Peptiden, wobei das Concatemer wie in Anspruch 8 definiert ist, wobei das Verfahren umfasst
(a) Ligieren eines wie in Anspruch 1(a) definierten Peptids als die N-terminale Komponente mit einem wie in Anspruch 1(b) oder 3 definierten Peptid als die C-terminale Komponente;
(b) Ligieren des in Schritt (a) erhaltenen Produkts als die C-terminale Komponente mit einem wie in Anspruch 1(b) oder 3 definierten Peptid als die N-terminale Komponente;
(c) Ligieren des in Schritt (b) erhaltenen Produkts als die C-terminale Komponente mit einem wie in Anspruch 1(a) definierten Peptid als die N-terminale Komponente;
(d) gegebenenfalls Wiederholen der Schritte (b) und (c) bis ein Concatemer der gewünschten Länge erhalten wird;
(e) Ligieren des in Schritt (c) bzw. (d) erhaltenen Produkts als die C-terminale Komponente mit einem wie in Anspruch 1(b) oder 3 definierten Peptid als die N-terminale Komponente; und
(f) Ligieren des in Schritt (e) erhaltenen Produkts als die C-terminale Komponente mit einem wie in Anspruch 1(a) definierten Peptid als die N-terminale Komponente.

12. Verfahren zum Produzieren einer Sammlung von Concatemeren von Peptiden, wobei das Concatemer wie in Anspruch 8 definiert ist, wobei das Verfahren umfasst
(a) Ligieren eines oder mehrerer wie in Anspruch 1(a) definierter Peptide als die N-terminalen Komponenten mit einem oder mehreren wie in Anspruch 1(b) oder 3 definierten Peptiden oder mit einer wie in Anspruch 4 definierten Sammlung als die C-terminalen Komponenten;
(b) Ligieren der in Schritt (a) erhaltenen Produkte als die C-terminalen Komponenten mit einem oder mehreren wie in Anspruch 1(b) oder 3 definierten Peptide oder mit einer wie in Anspruch 4 definierten Sammlung als die N-terminalen Komponenten;
(c) Ligieren der in Schritt (b) erhaltenen Produkte als die C-terminalen Komponenten mit einem oder mehreren der wie in Anspruch 1(a) definierten Peptide als die N-terminalen Komponenten;
(d) gegebenenfalls Wiederholen der Schritte (b) und (c) bis ein Concatemer der gewünschten Länge erhalten wird;
(e) Ligieren der Produkte, die in Schritt (c) bzw. (d) erhalten wurden, als die C-terminalen Komponenten mit einem oder mehreren wie in Anspruch 1(b) oder 3 definierten Peptide oder einer wie in Anspruch 4 definierten Sammlung als die N-terminalen Komponenten; und
(f) Ligieren der in Schritt (e) erhaltenen Produkte als die C-terminalen Komponenten mit einem oder mehreren der wie in Anspruch 1(a) definierten Peptide als die N-terminalen Komponenten.

13. Verfahren nach einem der Ansprüche 9 bis 12, wobei
(i) der C-terminale Rest der N-terminalen Komponente(n) einen Thioester trägt;
(ii) der N-terminale Rest der C-terminalen Komponente(n) Cys ist; und
(iii) Ligieren unter Bedingungen durchgeführt wird, die das Ausbilden einer Peptidbindung zwischen einem C-terminalen Thioester und einer N-terminalen Thiol-enthaltenden Aminosäure erlauben.

14. Kit umfassend oder bestehend aus
(a) ein(em) oder mehrere(n) Peptide(n) wie in einem der Ansprüche 1 (a) oder 2 definiert; und
(b) ein(em) oder mehrere(n) Peptide(n) wie in einem der Ansprüche 1 (b), 3, 5 oder 7 definiert und/oder eine(r) Sammlung nach einem der Ansprüche 4 bis 7.

15. Kit umfassend oder bestehend aus
(a) eine(r) Sammlung von Peptiden, wobei jedes Peptid aus der Sequenz besteht, wie sie in einer der SEQ ID Nr.: 73 bis 81 dargestellt ist, oder eine(r) Sammlung, die mit dem Verfahren gemäß Anspruch 10 erhältlich ist; und/oder
(b) eine(r) Sammlung von Concatemeren, wobei jedes Concatemer wie in Anspruch 8 definiert ist, oder eine(r) Sammlung, die mit dem Verfahren nach Anspruch 12 erhältlich ist.

16. Fester Träger mit einem oder mehreren Peptiden, einem oder mehreren Concatemeren und/oder einer oder mehreren Sammlungen, die auf der Oberfläche des festen Trägers immobilisiert sind;
wobei das Peptid/die Peptide aus der Sequenz bestehen, die in einer der SEQ ID Nr.: 73 bis 81 dargestellt ist;
das Concatemer/die Concatemere wie in Anspruch 8 definiert sind; und
die Sammlungen eine oder mehrere Sammlungen von Peptiden umfassen oder aus diesen bestehen, wobei jedes Peptid aus der Sequenz besteht, wie sie in einer der SEQ ID Nr.: 73 bis 81 dargestellt ist, oder die eine oder die mehreren Sammlungen von Peptiden mit dem Verfahren gemäß Anspruch 10 erhältlich sind; und/oder eine oder mehrere Sammlungen von Concatemeren, wobei jedes Concatemer wie in Anspruch 8 definiert ist, oder die eine oder die mehreren Sammlungen von Concatemeren mit dem Verfahren gemäß Anspruch 12 erhältlich sind.

## Revendications

1. Utilisation d'un peptide constitué de la séquence présentée dans l'une quelconque des
(a) SEQ ID N^{os} : 1 à 3 ; et/ou
(b) SEQ ID N° : 7
dans la synthèse d'un peptide, d'un polypeptide ou d'une protéine, ledit peptide, polypeptide ou ladite protéine comprenant ou étant constitué(e) d'un ou de plusieurs domaines à doigt de zinc.

2. Utilisation selon la revendication 1 (a), dans laquelle le résidu N-terminal de la séquence de l'une quelconque des SEQ ID N^{os}: 1 à 3 est Cys.

3. Peptide constitué de la séquence présentée dans l'une quelconque des SEQ ID N^{os} : 6 ou 8.

4. Collection de peptides, la collection comprenant ou étant constituée d'une pluralité de peptides différents, chaque peptide étant constitué d'une séquence telle que présentée dans SEQ ID N^{os} : 1, 2, 3, 6, 7 ou 8.

5. Utilisation selon la revendication 1 (b), peptide selon la revendication 3 ou collection de peptides selon la revendication 4, dans laquelle/lequel le résidu N-terminal de la séquence de l'une quelconque des SEQ ID N^{os}: 6 à 8 dans ledit peptide ou dans au moins un desdits peptides est Cys.

6. Collection selon la revendication 5, dans laquelle la collection comprend ou est constituée de tous les peptides constitués des séquences présentées dans SEQ ID N^{os} : 9 à 72.

7. Utilisation selon les revendications 1, 2 ou 5, peptide selon les revendications 3 ou 5, ou collection selon l'une quelconque des revendications 4 à 6, dans laquelle/lequel le résidu C-terminal dudit peptide ou les résidus C-terminaux d'au moins un peptide de ladite collection porte(nt) un thioester.

8. Concatémère de peptides, chaque peptide étant constitué de la séquence présentée dans l'une quelconque des SEQ ID N^{os} : 73 à 81.

9. Procédé de production d'un peptide constitué de la séquence présentée dans l'une quelconque des SEQ ID N^{os}: 73 à 81, ledit procédé comprenant l'étape de ligature d'un peptide constitué de la séquence présentée dans l'une quelconque des SEQ ID N^{os}: 1 à 3 à titre de composant N-terminal avec un peptide constitué de la séquence présentée dans l'une quelconque des SEQ ID N^{os} : 6 à 8 à titre de composant C-terminal.

10. Procédé de production d'une collection de peptides, chaque peptide étant constitué de la séquence présentée dans l'une quelconque des SEQ ID N^{os}: 73 à 81, le procédé comprenant l'étape de ligature d'un ou de plusieurs peptides tels que définis dans la revendication 1(a) à titre de composant(s) N-terminal(aux) avec un ou plusieurs peptides tels que définis dans la revendication 1 (b) ou 3 ou avec une collection telle que définie dans la revendication 4 à titre de composant(s) C-terminal(aux).

11. Procédé de production d'un concatémère de peptides, le concatémère étant tel que défini dans la revendication 8, le procédé comprenant :
(a) la ligature d'un peptide tel que défini dans la revendication 1 (a) à titre de composant N-terminal avec un peptide tel que défini dans la revendication 1 (b) ou 3 à titre de composant C-terminal ;
(b) la ligature du produit obtenu à l'étape (a) à titre de composant C-terminal avec un peptide tel que défini dans la revendication 1 (b) ou 3 à titre de composant N-terminal ;
(c) la ligature du produit obtenu à l'étape (b) à titre de composant C-terminal avec un peptide tel que défini dans la revendication 1(a) à titre de composant N-terminal ;
(d) éventuellement, la répétition des étapes (b) et (c) jusqu'à ce qu'un concatémère de longueur souhaitée soit obtenu ;
(e) la ligature du produit obtenu à l'étape (c) ou (d), respectivement, à titre de composant C-terminal, avec un peptide tel que défini dans la revendication 1 (b) ou 3 à titre de composant N-terminal ; et
(f) la ligature du produit obtenu à l'étape (e) à titre de composant C-terminal avec un peptide tel que défini dans la revendication 1 (a) à titre de composant N-terminal.

12. Procédé de production d'une collection de concatémères de peptides, le concatémère étant tel que défini dans la revendication 8, le procédé comprenant
(a) la ligature d'un ou de plusieurs peptides tel que défini dans la revendication 1 (a) à titre de composants N-terminaux avec un ou plusieurs peptides tels que définis dans la revendication 1 (b) ou 3 ou avec une collection telle que définie dans la revendication 4 à titre de composants C-terminaux ;
(b) la ligature des produits obtenus à l'étape (a) à titre de composants C-terminaux avec un ou plusieurs peptides tels que définis dans la revendication 1 (b) ou 3 ou avec une collection telle que définie dans la revendication 4, respectivement, à titre de composants N-terminaux ;
(c) la ligature des produits obtenus à l'étape (b) à titre de composants C-terminaux avec un ou plusieurs peptides tels que définis dans la revendication 1(a) à titre de composants N-terminaux ;
(d) éventuellement, la répétition des étapes (b) et (c) jusqu'à ce qu'un concatémère de longueur souhaitée soit obtenu ;
(e) la ligature des produits obtenus à l'étape (c) ou (d), respectivement, à titre de composants C-terminaux, avec un ou plusieurs peptides tels que définis dans la revendication 1(b) ou 3 ou avec une collection selon la revendication 4, respectivement, à titre de composants N-terminaux ; et
(f) la ligature des produits obtenus à l'étape (e) à titre de composants C-terminaux avec un ou plusieurs peptides tels que définis dans la revendication 1 (a) à titre de composants N-terminaux.

13. Procédé selon l'une quelconque des revendications 9 à 12, dans lequel
(i) le résidu C-terminal du ou des composants N-terminaux porte un thioester ;
(ii) le résidu N-terminal du ou des composants C-terminaux est Cys ;
et
(iii) la ligature est réalisée dans des conditions permettant la formation d'une liaison peptidique entre un thioester C-terminal et un acide aminé N-terminal contenant un thiol.

14. Kit comprenant ou constitué de
(a) un ou plusieurs peptides tels que définis dans l'une quelconque des revendications 1 (a) ou 2 ; et
(b) un ou plusieurs peptides tels que définis dans l'une quelconque des revendications 1(b), 3, 5 ou 7 et/ou une collection selon l'une quelconque des revendications 4 à 7.

15. Kit comprenant ou constitué de
(a) une collection de peptides, chaque peptide étant constitué de la séquence présentée dans l'une quelconque des SEQ ID N^{os}: 73 à 81, ou une collection susceptible d'être obtenue par le procédé selon la revendication 10 ; et/ou
(b) une collection de concatémères, chaque concatémère étant tel que défini dans la revendication 8, ou une collection pouvant être obtenue par le procédé selon la revendication 12.

16. Support solide comportant un ou plusieurs peptides, un ou plusieurs concatémères, et/ou une ou plusieurs collection(s) étant immobilisée(s) sur la surface dudit support ;
ledit ou lesdits peptides étant constitués de la séquence présentée dans l'une quelconque des SEQ ID N^{os}: 73 à 81;
ledit ou lesdits concatémères étant tels que définis dans la revendication 8 ; et
lesdites collections étant constituées de, ou comprenant une ou plusieurs collections de peptides, chaque peptide étant constitué de la séquence présentée dans l'une quelconque des SEQ ID N^{os} : 73 à 81, ou la ou les collections de peptides étant susceptibles d'être obtenues par le procédé selon la revendication 10 ; et/ou une ou plusieurs collections de concatémères, chaque concatémère étant tel que défini dans la revendication 8, ou la ou les collections de concatémères étant susceptibles d'être obtenues par le procédé selon la revendication 12.


### SEQUENCE LISTING

<110> Fraunhofer-Gesellschaft zur Förderung der angewandten Forschung e.V.
<120> Means and methods for producing zinc fingers and concatemers thereof
<130> N3334 EP
<160> 81
<170> PatentIn version 3.3
<210> 1
   <211> 9
   <212> PRT
   <213> artificial sequence
<220>
   <221> source
   <223> /note="description of artificial sequence: first peptide"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<400> 1
   Cys Ala Cys Ala Ala Cys Ala Ala Ala
   1               5
<210> 2
   <211> 10
   <212> PRT
   <213> artificial sequence
<220>
   <221> source
   <223> /note="Description of artificial sequence: first peptide"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace-"Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace-"Arg" /replace="Ser" /replace="Thr" /replace="Val" /replace="Trp" /replace="Tyr"

<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace="Asp" /replace="Glu" /replace-"Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace-"Val" /replace="Trp" /replace="Tyr"
<400> 2
   Cys Ala Cys Ala Ala Ala Cys Ala Ala Ala
   1               5                   10
<210> 3
   <211> 11
   <212> PRT
   <213> artificial sequence
<220>
   <221> source
   <223> /note="Description of artificial sequence: first peptide"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace-"Thr" /replace="Val" /replace="Trp" /replace="Tyr"
<220>
   <221> variation
   <222> 10
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Cys /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 3
   Cys Ala Cys Ala Ala Ala Ala Cys Ala Ala Ala
   1               5                   10
<210> 4
   <211> 10
   <212> PRT
   <213> artificial sequence
<220>
   <221> source
   <223> /note="Description of artificial sequence: first peptide"
<400> 4
   Cys Met Cys Thr Ser Tyr Cys Gly Lys Arg
   1               5                   10
<210> 5
   <211> 10
   <212> PRT
   <213> artificial sequence
<220>
   <221> source
   <223> /note="Description of artificial sequence: TAT peptide"
<400> 5
   Gly Arg Lys Lys Arg Arg Gln Arg Arg Arg
   1               5                   10
<210> 6
   <211> 19
   <212> PRT
   <213> artificial sequence
<220>
   <221> source
   <223> /note="Description of artificial sequence: second peptide"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="val" /replace="Trp" /replace="Tyr"
<220>
   <221> variation
   <222> 3
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 6
   <223> /replace="Cys" /replace="Asp" /replace-"Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" Ireplace="Lys" Ireplace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Cys" /replace-"Asp" /replace="Glu" /replace="Phe" /replace="Gly" /replace="His" /replace="Ile" /replace-"Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace-"Gln" /replace="Arg" /replace="Ser" /replace="Thr" /replace="Val" /replace="Trp" /replace="Tyr"

<400> 6
   Cys Ala Ala Ala Ala Ala Leu Ala Ala His Ala Ala Ala His Ala Ala
   1               5                   10                  15
   Ala Ala Ala
<210> 7
   <211> 20
   <212> PRT
   <213> artificial sequence
<220>
   <221> source
   <223> /note="Description of artificial sequence: second peptide"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 3
   <223> /replace="Cys" /replace="Asp" /replace-"Glu" /replace="Phe" /replace-"Gly" /replace="His" Ireplace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace-"Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Cys" /replace="Asp" /replace-"Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 7
   Cys Ala Ala Ala Ala Ala Leu Ala Ala His Ala Ala Ala Ala His Ala
   1               5                   10                  15
   Ala Ala Ala Ala
               20
<210> 8
   <211> 21
   <212> PRT
   <213> artificial sequence
<220>
   <221> source
   <223> /note="Description of artificial sequence: second peptide"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 3
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace-"Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 8
<210> 9
   <211> 19
   <212> PRT
   <213> artificial sequence
<220>
   <221> source
   <223> /note="Description of artificial sequence: second peptide"

<400> 9
   Cys Thr Arg Ser Gln Asp Leu Gln Lys His Lys Arg Thr His Thr Gly
   1 5 10 15
   Glu Lys Pro
<210> 10
   <211> 19
   <212> PRT
   <213> artificial sequence
<220>
   <221> source
   <223> /note="Description of artificial sequence: second peptide"
<400> 10
   Cys Thr Gln Ser Ser Asn Leu Gln Asn His Lys Arg Thr His Thr Gly
   1 5 10 15
   Glu Lys Pro
<210> 11
   <211> 19
   <212> PRT
   <213> artificial sequence
<220>
   <221> source
   <223> /note="Description of artificial sequence: second.peptide"
<400> 11
   Cys Thr Arg Ser Ser His Leu Gln Lys His Lys Arg Thr His Thr Gly
   1 5 10 15
   Glu Lys Pro
<210> 12
   <211> 19
   <212> PRT
   <213> artificial sequence
<220>
   <221> source
   <223> /note="Description of artificial sequence: second peptide"
<400> 12
   Cys Thr Gln Ser Ser His Leu Gln Lys His Lys Arg Thr His Thr Gly
   1 5 10 15
   Glu Lys Pro
<210> 13
   <211> 19
   <212> PRT
   <213> artificial sequence
<220>
   <221> source
   <223> /note="Description of artificial sequence: second peptide"
<400> 13
   Cys Thr Asp Ser Ser His Leu Gln Lys His Lys Arg Thr His Thr Gly
   1               5                   10                  15
   Glu Lys Pro
<210> 14
   <211> 19
   <212> PRT
   <213> artificial sequence
<220>
   <221> source
   <223> /note="Description of artificial sequence: second peptide"
<400> 14
   Cys Thr Glu Ser Ser His Leu Gln Lys His Lys Arg Thr His Thr Gly
   1               5                   10                  15
   Glu Lys Pro
<210> 15
   <211> 19
   <212> PRT
   <213> artificial sequence
<220>
   <221> source
   <223> /note="Description of artificial sequence: second peptide"
<400> 15
   Cys Thr Arg Ser Ser His Leu Gln Asn His Lys Arg Thr His Thr Gly
   1               5                   10                  15
   Glu Lys Pro
<210> 16
   <211> 19
   <212> PRT
   <213> artificial sequence
<220>
   <221> source
   <223> /note="Description of artificial sequence: second peptide"
<400> 16
   Cys Thr Arg Ser Ser His Leu Gln Thr His Lys Arg Thr His Thr Gly
   1 5 10 15
   Glu Lys Pro
<210> 17
   <211> 19
   <212> PRT
   <213> artificial sequence
<220>
   <221> source
   <223> /note="Description of artificial sequence: second peptide"
<400> 17
   Cys Thr Arg Ser Ser His Leu Gln Glu His Lys Arg Thr His Thr Gly
   1 5 10 15
   Glu Lys Pro
<210> 18
   <211> 19
   <212> PRT
   <213> artificial sequence
<220>
   <221> source
   <223> /note="Description of artificial sequence: second peptide"
<400> 18
   Cys Thr Arg Ser Gly Asn Leu Gln Asn His Lys Arg Thr His Thr Gly
   1 5 10 15
   Glu Lys Pro
<210> 19
   <211> 19
   <212> PRT
   <213> artificial sequence
<220>
   <221> source
   <223> /note="Description of artificial sequence: second peptide"
<400> 19
   Cys Thr Glu Ser Gly Asn Leu Gln Asn His Lys Arg Thr His Thr Gly
   1 5 10 15
   Glu Lys Pro
<210> 20
   <211> 19
   <212> PRT
   <213> artificial sequence
<220>
   <221> source
   <223> /note="Description of artificial sequence: second peptide"
<400> 20
   Cys Thr Asp Ser Gly Asn Leu Gln Asn His Lys Arg Thr His Thr Gly
   1               5                   10                  15
   Glu Lys Pro
<210> 21
   <211> 19
   <212> PRT
   <213> artificial sequence
<220>
   <221> source
   <223> /note="Description of artificial sequence: second peptide"
<400> 21
   Cys Thr Gln Ser Gly Asn Leu Gln Lys His Lys Arg Thr His Thr Gly
   1               5                   10                  15
   Glu Lys Pro
<210> 22
   <211> 19
   <212> PRT
   <213> artificial sequence
<220>
   <221> source
   <223> /note="Description of artificial sequence: second peptide"
<400> 22
   Cys Thr Gln Ser Gly Asn Leu Gln Thr His Lys Arg Thr His Thr Gly
   1               5                   10                  15
   Glu Lys Pro
<210> 23
   <211> 19
   <212> PRT
   <213> artificial sequence
<220>
   <221> source
   <223> /note="Description of artificial sequence: second peptide"
<400> 23
   Cys Thr Gln Ser Gly Asn Leu Gln Glu His Lys Arg Thr His Thr Gly
   1 5 10 15
   Glu Lys Pro
<210> 24
   <211> 19
   <212> PRT
   <213> artificial sequence
<220>
   <221> source
   <223> /note="Description of artificial sequence: second peptide"
<400> 24
   Cys Thr Glu Ser Asp Ser Leu Gln Thr His Lys Arg Thr His Thr Gly
   1 5 10 15
   Glu Lys Pro
<210> 25
   <211> 19
   <212> PRT
   <213> artificial sequence
<220>
   <221> source
   <223> /note="Description of artificial sequence: second peptide"
<400> 25
   Cys Thr Arg Ser Asp Ser Leu Gln Thr His Lys Arg Thr His Thr Gly
   1 5 10 15
   Glu Lys Pro
<210> 26
   <211> 19
   <212> PRT
   <213> artificial sequence
<220>
   <221> source
   <223> /note="Description of artificial sequence: second peptide"

<400> 26
   Cys Thr Gln Ser Asp Ser Leu Gln Thr His Lys Arg Thr His Thr Gly
   1 5 10 15
   Glu Lys Pro
<210> 27
   <211> 19
   <212> PRT
   <213> artificial sequence
<220>
   <221> source
   <223> /note="Description of artificial sequence: second peptide"
<400> 27
   Cys Thr Asp Ser Asp Ser Leu Gln Thr His Lys Arg Thr His Thr Gly
   1 5 10 15
   Glu Lys Pro
<210> 28
   <211> 19
   <212> PRT
   <213> artificial sequence
<220>
   <221> source
   <223> /note="Description of artificial sequence: second peptide"
<400> 28
   Cys Thr Glu Ser Asp Ser Leu Gln Lys His Lys Arg Thr His Thr Gly
   1 5 10 15
   Glu Lys Pro
<210> 29
   <211> 19
   <212> PRT
   <213> artificial sequence
<220>
   <221> source
   <223> /note="Description of artificial sequence: second peptide"
<400> 29
   Cys Thr Glu Ser Asp Ser Leu Gln Asn His Lys Arg Thr His Thr Gly
   1 5 10 15
   Glu Lys Pro
<210> 30
   <211> 19
   <212> PRT
   <213> artificial sequence
<220>
   <221> source
   <223> /note="Description of artificial sequence: second peptide"
<400> 30
   Cys Thr Glu Ser Asp Ser Leu Gln Glu His Lys Arg Thr His Thr Gly
   1 5 10 15
   Glu Lys Pro
<210> 31
   <211> 19
   <212> PRT
   <213> artificial sequence
<220>
   <221> source
   <223> /note="Description of artificial sequence: second peptide"
<400> 31
   Cys Thr Asp Ser Gln Asp Leu Gln Glu His Lys Arg Thr His Thr Gly
   1 5 10 15
   Glu Lys Pro
<210> 32
   <211> 19
   <212> PRT
   <213> artificial sequence
<220>
   <221> source
   <223> /note="Description of artificial sequence: second peptide"
<400> 32
   Cys Thr Arg Ser Gln Asp Leu Gln Glu His Lys Arg Thr His Thr Gly
   1 5 10 15
   Glu Lys Pro
<210> 33
   <211> 19
   <212> PRT
   <213> artificial sequence
<220>
   <221> source
   <223> /note="Description of artificial sequence: second peptide"
<400> 33
   Cys Thr Gln Ser Gln Asp Leu Gln Glu His Lys Arg Thr His Thr Gly
   1 5 10 15
   Glu Lys Pro
<210> 34
   <211> 19
   <212> PRT
   <213> artificial sequence
<220>
   <221> source
   <223> /note="Description of artificial sequence: second peptide"
<400> 34
   Cys Thr Glu Ser Gln Asp Leu Gln Glu His Lys Arg Thr His Thr Gly
   1 5 10 15
   Glu Lys Pro
<210> 35
   <211> 19
   <212> PRT
   <213> artificial sequence
<220>
   <221> source
   <223> /note="Description of artificial sequence: second peptide"
<400> 35
   Cys Thr Asp Ser Gln Asp Leu Gln Lys His Lys Arg Thr His Thr Gly
   1 5 10 15
   Glu Lys Pro
<210> 36
   <211> 19
   <212> PRT
   <213> artificial sequence
<220>
   <221> source
   <223> /note="Description of artificial sequence: second peptide"
<400> 36
   Cys Thr Asp Ser Gln Asp Leu Gln Asn His Lys Arg Thr His Thr Gly
   1 5 10 15
   Glu Lys Pro
<210> 37
   <211> 19
   <212> PRT
   <213> artificial sequence
<220>
   <221> source
   <223> /note="Description of artificial sequence: second peptide"
<400> 37
   Cys Thr Asp Ser Gln Asp Leu Gln Thr His Lys Arg Thr His Thr Gly
   1 5 10 15
   Glu Lys Pro
<210> 38
   <211> 19
   <212> PRT
   <213> artificial sequence
<220>
   <221> source
   <223> /note="Description of artificial sequence: second peptide"
<400> 38
   Cys Thr Gln Ser Gln Asp Leu Gln Lys His Lys Arg Thr His Thr Gly
   1 5 10 15
   Glu Lys Pro
<210> 39
   <211> 19
   <212> PRT
   <213> artificial sequence
<220>
   <221> source
   <223> /note="Description of artificial sequence: second peptide"
<400> 39
   Cys Thr Glu Ser Gln Asp Leu Gln Lys His Lys Arg Thr His Thr Gly
   1 5 10 15
   Glu Lys Pro
<210> 40
   <211> 19
   <212> PRT
   <213> artificial sequence
<220>
   <221> source
   <223> /note="Description of artificial sequence: second peptide"
<400> 40
   Cys Thr Arg Ser Gly Asn Leu Gln Lys His Lys Arg Thr His Thr Gly
   1 5 10 15
   Glu Lys Pro
<210> 41
   <211> 19
   <212> PRT
   <213> artificial sequence
<220>
   <221> source
   <223> /note="Description of artificial sequence: second peptide"
<400> 41
   Cys Thr Glu Ser Gly Asn Leu Gln Lys His Lys Arg Thr His Thr Gly
   1 5 10 15
   Glu Lys Pro
<210> 42
   <211> 19
   <212> PRT
   <213> artificial sequence
<220>
   <221> source
   <223> /note="Description of artificial sequence: second peptide"
<400> 42
   Cys Thr Asp Ser Gly Asn Leu Gln Lys His Lys Arg Thr His Thr Gly
   1 5 10 15
   Glu Lys Pro
<210> 43
   <211> 19
   <212> PRT
   <213> artificial sequence
<220>
   <221> source
   <223> /note="Description of artificial sequence: second peptide"

<400> 43
   Cys Thr Arg Ser Asp Ser Leu Gln Lys His Lys Arg Thr His Thr Gly
   1 5 10 15
   Glu Lys Pro
<210> 44
   <211> 19
   <212> PRT
   <213> artificial sequence
<220>
   <221> source
   <223> /note="Description of artificial sequence: second peptide"
<400> 44
   Cys Thr Gln Ser Asp Ser Leu Gln Lys His Lys Arg Thr His Thr Gly
   1 5 10 15
   Glu Lys Pro
<210> 45
   <211> 19
   <212> PRT
   <213> artificial sequence
<220>
   <221> source
   <223> /note="Description of artificial sequence: second peptide"
<400> 45
   Cys Thr Asp Ser Asp Ser Leu Gln Lys His Lys Arg Thr His Thr Gly
   1 5 10 15
   Glu Lys Pro
<210> 46
   <211> 19
   <212> PRT
   <213> artificial sequence
<220>
   <221> source
   <223> /note="Description of artificial sequence: second peptide"
<400> 46
   Cys Thr Gln Ser Ser His Leu Gln Asn His Lys Arg Thr His Thr Gly
   1 5 10 15
   Glu Lys Pro
<210> 47
   <211> 19
   <212> PRT
   <213> artificial sequence
<220>
   <221> source
   <223> /note="Description of artificial sequence: second peptide"
<400> 47
   Cys Thr Gln Ser Gln Asp Leu Gln Asn His Lys Arg Thr His Thr Gly
   1 5 10 15
   Glu Lys Pro
<210> 48
   <211> 19
   <212> PRT
   <213> artificial sequence
<220>
   <221> source
   <223> /note="Description of artificial sequence: second peptide"
<400> 48
   Cys Thr Gln Ser Asp Ser Leu Gln Asn His Lys Arg Thr His Thr Gly
   1 5 10 15
   Glu Lys Pro
<210> 49
   <211> 19
   <212> PRT
   <213> artificial sequence
<220>
   <221> source
   <223> /note="Description.of artificial sequence: second peptide"
<400> 49
   Cys Thr Glu Ser Gln Asp Leu Gln Asn His Lys Arg Thr His Thr Gly
   1 5 10 15
   Glu Lys Pro
<210> 50
   <211> 19
   <212> PRT
   <213> artificial sequence
<220>
   <221> source
   <223> /note="Description of artificial sequence: second peptide"
<400> 50
   Cys Thr Arg Ser Gln Asp Leu Gln Asn His Lys Arg Thr His Thr Gly
   1 5 10 15
   Glu Lys Pro
<210> 51
   <211> 19
   <212> PRT
   <213> artificial sequence
<220>
   <221> source
   <223> /note="Description of artificial sequence: second peptide"
<400> 51
   Cys Thr Asp Ser Asp Ser Leu Gln Asn His Lys Arg Thr His Thr Gly
   1 5 10 15
   Glu Lys Pro
<210> 52
   <211> 19
   <212> PRT
   <213> artificial sequence
<220>
   <221> source
   <223> /note="Description of artificial sequence: second peptide"
<400> 52
   Cys Thr Asp Ser Ser His Leu Gln Asn His Lys Arg Thr His Thr Gly
   1 5 10 15
   Glu Lys Pro
<210> 53
   <211> 19
   <212> PRT
   <213> artificial sequence
<220>
   <221> source
   <223> /note="Description of artificial sequence: second peptide"
<400> 53
   Cys Thr Glu Ser Ser His Leu Gln Asn His Lys Arg Thr His Thr Gly
   1 5 10 15
   Glu Lys Pro
<210> 54
   <211> 19
   <212> PRT
   <213> artificial sequence
<220>
   <221> source
   <223> /note="Description of artificial sequence: second peptide"
<400> 54
   Cys Thr Arg Ser Asp Ser Leu Gln Asn His Lys Arg Thr His Thr Gly
   1 5 10 15
   Glu Lys Pro
<210> 55
   <211> 19
   <212> PRT
   <213> artificial sequence
<220>
   <221> source
   <223> /note="Description of artificial sequence: second peptide"
<400> 55
   Cys Thr Glu Ser Gly Asn Leu Gln Thr His Lys Arg Thr His Thr Gly
   1 5 10 15
   Glu Lys Pro
<210> 56
   <211> 19
   <212> PRT
   <213> artificial sequence
<220>
   <221> source
   <223> /note="Description of artificial sequence: second peptide"
<400> 56
   Cys Thr Glu Ser Ser His Leu Gln Thr His Lys Arg Thr His Thr Gly
   1 5 10 15
   Glu Lys Pro
<210> 57
   <211> 19
   <212> PRT
   <213> artificial sequence
<220>
   <221> source
   <223> /note="Description of artificial sequence: second peptide"
<400> 57
   Cys Thr Glu Ser Gln Asp Leu Gln Thr His Lys Arg Thr His Thr Gly
   1 5 10 15
   Glu Lys Pro
<210> 58
   <211> 19
   <212> PRT
   <213> artificial sequence
<220>
   <221> source
   <223> /note="Description of artificial sequence: second peptide"
<400> 58
   Cys Thr Gln Ser Gln Asp Leu Gln Thr His Lys Arg Thr His Thr Gly
   1 5 10 15
   Glu Lys Pro
<210> 59
   <211> 19
   <212> PRT
   <213> artificial sequence
<220>
   <221> source
   <223> /note="Description of artificial sequence: second peptide"
<400> 59
   Cys Thr Arg Ser Gln Asp Leu Gln Thr His Lys Arg Thr His Thr Gly
   1 5 10 15
   Glu Lys Pro
<210> 60
   <211> 19
   <212> PRT
   <213> artificial sequence
<220>
   <221> source
   <223> /note="Description of artificial sequence: second peptide"

<400> 60
   Cys Thr Asp Ser Gly Asn Leu Gln Thr His Lys Arg Thr His Thr Gly
   1               5                   10                  15
   Glu Lys Pro
<210> 61
   <211> 19
   <212> PRT
   <213> artificial sequence
<220>
   <221> source
   <223> /note="Description of artificial sequence: second peptide"
<400> 61
   Cys Thr Asp Ser Ser His Leu Gln Thr His Lys Arg Thr His Thr Gly
   1               5                   10                  15
   Glu Lys Pro
<210> 62
   <211> 19
   <212> PRT
   <213> artificial sequence
<220>
   <221> source
   <223> /note="Description of artificial sequence: second peptide"
<400> 62
   Cys Thr Arg Ser Gly Asn Leu Gln Thr His Lys Arg Thr His Thr Gly
   1               5                   10                  15
   Glu Lys Pro
<210> 63
   <211> 19
   <212> PRT
   <213> artificial sequence
<220>
   <221> source
   <223> /note="Description of artificial sequence: second peptide"
<400> 63
<210> 64
   <211> 19
   <212> PRT
   <213> artificial sequence
<220>
   <221> source
   <223> /note="Description of artificial sequence: second peptide"
<400> 64
   Cys Thr Asp Ser Gly Asn Leu Gln Glu His Lys Arg Thr His Thr Gly
   1 5 10 15
   Glu Lys Pro
<210> 65
   <211> 19
   <212> PRT
   <213> artificial sequence
<220>
   <221> source
   <223> /note="Description of artificial sequence: second peptide"
<400> 65
   Cys Thr Asp Ser Asp Ser Leu Gln Glu His Lys Arg Thr His Thr Gly
   1 5 10 15
   Glu Lys Pro
<210> 66
   <211> 19
   <212> PRT
   <213> artificial sequence
<220>
   <221> source
   <223> /note="Description of artificial sequence: second peptide"
<400> 66
   Cys Thr Asp Ser Ser His Leu Gln Glu His Lys Arg Thr His Thr Gly
   1 5 10 15
   Glu Lys Pro
<210> 67
   <211> 19
   <212> PRT
   <213> artificial sequence
<220>
   <221> source
   <223> /note="Description of artificial sequence: second peptide"
<400> 67
   Cys Thr Gln Ser Ser His Leu Gln Glu His Lys Arg Thr His Thr Gly
   1               5                   10                  15
   Glu Lys Pro
<210> 68
   <211> 19
   <212> PRT
   <213> artificial sequence
<220>
   <221> source
   <223> /note="Description of artificial sequence: second peptide"
<400> 68
   Cys Thr Glu Ser Ser His Leu Gln Glu His Lys Arg Thr His Thr Gly
   1               5                   10                  15
   Glu Lys Pro
<210> 69
   <211> 19
   <212> PRT
   <213> artificial sequence
<220>
   <221> source
   <223> /note="Description of artificial sequence: second peptide"
<400> 69
   Cys Thr Arg Ser Gly Asn Leu Gln Glu His Lys Arg Thr His Thr Gly
   1               5                   10                  15
   Glu Lys Pro
<210> 70
   <211> 19
   <212> PRT
   <213> artificial sequence
<220>
   <221> source
   <223> /note="Description of artificial sequence: second peptide"
<400> 70
   Cys Thr Glu Ser Gly Asn Leu Gln Glu His Lys Arg Thr His Thr Gly
   1               5                   10                  15
   Glu Lys Pro
<210> 71
   <211> 19
   <212> PRT
   <213> artificial sequence
<220>
   <221> source
   <223> /note="Description of artificial sequence: second peptide"
<400> 71
   Cys Thr Gln Ser Asp Ser Leu Gln Glu His Lys Arg Thr His Thr Gly
   1               5                   10                  15
   Glu Lys Pro
<210> 72
   <211> 19
   <212> PRT
   <213> artificial sequence
<220>
   <221> source
   <223> /note="Description of artificial sequence: second peptide"
<400> 72
   Cys Thr Arg Ser Asp Ser Leu Gln Glu His Lys Arg Thr His Thr Gly
   1               5                   10                  15
   Glu Lys Pro
<210> 73
   <211> 28
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace-"Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Leu" /replace="Ile" /replace="Val" /replace="Met" /replace="Phe" /zeplace="Tyr" /replace="Trp"
<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace-"Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" Ireplace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" Ireplace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 25
   <223> /replace="Cys"
   /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 73
   Cys Ala Cys Ala Ala Cys Ala Ala Ala Cys Ala Ala Ala Ala Ala Leu
   1               5                   10                  15
   Ala Ala His Ala Ala Ala His Ala Ala Ala Ala Ala
               20                  25
<210> 74
   <211> 29
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replaee="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Leu" /replace="Ile" /replace="Val" /replace="Met" /replace="Phe" /replace="Tyr" /replace="Trp"
<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro /replace="Gln" /replace="Arg" /replace="Ser" /replace-"Thr" /replace="Val" /replace="Trp" /replace="Tyr"

<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu"
   /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 25
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 74
   Cys Ala Cys Ala Ala Cys Ala Ala Ala Cys Ala Ala Ala Ala Ala Leu
   1               5                   10                  15
   Ala Ala His Ala Ala Ala Ala His Ala Ala Ala Ala Ala
               20                  25
<210> 75
   <211> 30
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Leu" /replace="Ile" /replace="Val" /replace="Met" /replace="Phe" /replace="Tyr" /replace="Trp"
<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys"
   /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace-"Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 30
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 75
   Cys Ala Cys Ala Ala Cys Ala Ala Ala Cys Ala Ala Ala Ala Ala Leu
   1               5                   10                  15
   Ala Ala His Ala Ala Ala Ala Ala His Ala Ala Ala Ala Ala
               20                  25                  30
<210> 76
   <211> 29
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Leu" /replace="Ile" /replace="Val" /replace="Met" /replace="Phe" /replace="Tyr" /replace="Trp"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 25
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 76
   Cys Ala Cys Ala Ala Ala Cys Ala Ala Ala Cys Ala Ala Ala Ala Ala
   1               5                   10                  15
   Leu Ala Ala His Ala Ala Ala His Ala Ala Ala Ala Ala
               20                  25
<210> 77
   <211> 30
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace-"Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Leu" /replace="Ile" /replace="Val" /replace="Met" /replace="Phe" /replace="Tyr" /replace="Trp"

<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace-"Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace-"Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace-"Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 30
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 77
   Cys Ala Cys Ala Ala Ala Cys Ala Ala Ala Cys Ala Ala Ala Ala Ala
   1               5                   10                  15
   Leu Ala Ala His Ala Ala Ala Ala His Ala Ala Ala Ala Ala
               20                  25                  30
<210> 78
   <211> 31
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"

<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace-"Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 8
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 11
   <223> /replace="Leu" /replace="Ile" /replace="Val" /replace="Met" /replace="Phe" /replace="Tyr" /replace="Trp"
<220>
   <221> variation
   <222> 12
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 18
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 21
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation .
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 25
   <223> /replace="Cys" /replace="Asp" /replace="Glu" - /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 30
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 31
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 78
   Cys Ala Cys Ala Ala Ala Cys Ala Ala Ala Cys Ala Ala Ala Ala Ala
   1               5                   10                  15
   Leu Ala Ala His Ala Ala Ala Ala Ala His Ala Ala Ala Ala Ala
               20                  25                  30
<210> 79
   <211> 30
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace-"Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace-"Ser" /replace="Thr" /replace="Val" /replace="Trp" /replace="Tyr"
<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace-"Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Leu" /replace="Ile" /replace="Val" /replace="Met" /replace="Phe" /replace="Tyr" /replace="Trp"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace-"Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace-"Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 30
   <223> /replace="Cys" /replace="Asp" /replace="Glu" /replace="Phe" /replace="Gly" /replace="His" /replace="Ile" /replace="Lys" /replace-"Leu" /replace="Met" /replace="Asn" /replace="Pro" /replace="Gln" /replace="Arg" /replace="Ser" /replace="Thr" /replace="Val" /replace="Trp" /replace="Tyr"
<400> 79
   Cys Ala Cys Ala Ala Ala Ala Cys Ala Ala Ala Cys Ala Ala Ala Ala
   1               5                   10                  15
   Ala Leu Ala Ala His Ala Ala Ala His Ala Ala Ala Ala Ala
               20                  25                  30
<210> 80
   <211> 31
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace-"Met" /replace="Phe" /replace="Tyr" /replace="Trp"
<220>
   <221> variation
   <222> 2
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="ASp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" %replace="Thr" /replace="Val" /replace="Trp" /replace="Tyr"
<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Leu" /replace="Ile" /replace="Val" /replace="Met" /replace="Phe" /replace="Tyr" /replace="Trp"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace='"Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 25
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 27
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 30
   <223> /replace="Cys" /replace="Asp" /replace="Glu" /replace="Phe" /replace="Gly" /replace="His" /replace="Ile" /replace="Lys" /replace="Leu" /replace="Met /replace="Asn" /replace="Pro" /replace="Gln" /replace="Arg" /replace="Ser" /replace="Thr" /replace="Val" /replace="Trp" /replace="Tyr"
<220>
   <221> variation
   <222> 31
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 80
   Cys Ala Cys Ala Ala Ala Ala Cys Ala Ala Ala Cys Ala Ala Ala Ala
   1               5                   10                  15
   Ala Leu Ala Ala His Ala Ala Ala Ala His Ala Ala Ala Ala Ala
               20                  25                  30
<210> 81
   <211> 32
   <212> PRT
   <213> artificial sequence
<220>
   <221> source
   <223> /note="Description of artificial sequence: zinc finger domain"
<220>
   <221> variation
   <222> 1
   <223> /replace="Leu" /replace="Ile" /replace="Val" /replace="Met" /replace="Phe" /replace="Tyr" /replace="Trp"
<220>
   <221> variation
   <222> 2
   <223> /replace="Cys" 118 /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 4
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 5
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 6
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 7
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 9
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 10
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"

<220>
   <221> variation
   <222> 11
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 12
   <223> /replace="Leu" /replace="Ile" /replace="Val" /replace="Met" /replace="Phe" /replace="Tyr" /replace="Trp"
<220>
   <221> variation
   <222> 13
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 14
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 15
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 16
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 17
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 19
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 20
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 22
   <223> /replace="Cys" /replace="Asp" /replace="Glu" /replace="Phe" /replace="Gly" /replace-"His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace-"Tyr"
<220>
   <221> variation
   <222> 23
   <223> /replace="Cys" /replace="Asp" /replace="Glu" /replace="Phe" /replace="Gly" /replace="His" /replace="Ile" /replace="Lys" /replace="Leu" /replace="Met" /replace="Asn /replace="Pro" /replace="Gln" /replace="Arg" /replace="Ser" /replace="Thr" /replace="Val" /replace="Trp" /replace="Tyr"
<220>
   <221> variation
   <222> 24
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 25
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 26
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 28
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 29
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 30
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 31
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<220>
   <221> variation
   <222> 32
   <223> /replace="Cys" /replace="Asp" /replace="Glu" /replace="Phe" /replace="Gly" /replace="His" /replace="Ile" /replace="Lys" /replace="Leu" /replace="Met" /replace="Asn" /replace="Pro" /replace="Gln" /replace="Arg" /replace="Ser" /replace="Thr" /replace="Val" /replace="Trp" /replace="Tyr"
<400> 81
   Cys Ala Cys Ala Ala Ala Ala Cys Ala Ala Ala Cys Ala Ala Ala Ala
   1               5                   10                  15
   Ala Leu Ala Ala His Ala Ala Ala Ala Ala His Ala Ala Ala Ala Ala
               20                  25                  30